# EUROPEAN PATENT SPECIFICATION

(11) **EP 2 791 884 B1**
(45) Date of publication and mention of the grant of the patent: **24.10.2018**
(21) Application number: 12806383.1
(22) Date of filing: 06.12.2012
(51) Int. Cl.: G08B 21/24, G16H 40/20, G06Q 30/02, G06Q 50/22

(54) **SOAP BAR USAGE MONITORING**
ÜBERWACHUNG DER SEIFENSTÜCKVERWENDUNG
CONTRÔLE D'UTILISATION DE PAIN DE SAVON

(30) Priority: 12.12.2011 EP 11192970
(43) Date of publication of application: 22.10.2014
(73) Proprietor: Unilever PLC, London, Greater London EC4Y 0DY (GB); Unilever N.V., 3013 AL Rotterdam (NL)
(72) Inventor: BATES, Susan, Bebington Wirral Merseyside CH63 3JW (GB); ZILLMER, Ruediger, Bebington Wirral Merseyside CH63 3JW (GB)
(74) Representative: James, Helen Sarah
(86) International application number: PCT/EP2012/074700
(87) International publication number: WO 2013/087520

(56) References cited:
- WO-A1-2008/058817
- WO-A1-2010/028320

## Description

### Field of the Invention

The present invention relates to soap bar usage monitoring.

### Background of the Invention and Prior Art

The World Health Organisation (WHO) estimates that diarrhoea and respiratory infections are responsible for two-thirds of child deaths. The vast majority of child mortality occurs among the world's poorest populations in low- and middle-income countries.

Contamination of surfaces such as hands plays a key role in the spread of diarrhoeal pathogens and some respiratory infections. Appropriate use of soaps and surfactants for improving overall cleanliness would be an effective intervention for prevention, as opposed to treatment, of these infectious diseases. Soap does not necessarily kill germs, but helps to wash them away by improving wettability, reducing their numbers to below the critical infectious dose.

Recent research has shown that washing hands with soap at critical moments (particularly after contact with human excreta and before handling food) can cut diarrhoea risk by almost half. Another recent study indicates that hand washing with soap has the potential to reduce respiratory infections by about a third.

However, hand washing with soap is not common, though practice varies from country to country. The main reason given why rates of hand washing with soap are so low is that it is simply not a habit. The widely held belief is that if hands look clean, they are clean.

The food services and healthcare industries have developed various systems intended to address the problem of insufficient hand washing. Such systems typically involve complex hardware and elaborate electronics for sensing and monitoring the hand washing process. Therefore they are not suitable for use in a domestic setting by low-income consumers. Moreover they do not address the issue of educating consumers in the long term. Behaviour change and interventions to evoke change are essential to achieve this.

The ability to monitor various aspects of consumers' existing behaviour is key to developing effective intervention strategies and evoking behaviour change. What is needed is reliable, objective information about soap usage, in large populations. This enables the quantification of baseline soap usage, and therefore the effect of interventions.

WO 2008/058817 discloses a data logger in a soap bar, said data logger comprising a motion sensor and storing sensed motion data for computer analysis when sensed motion is above a threshold.

The present inventors have found that usage of a soap bar can be conveniently and unobtrusively monitored by embedding a logger device incorporating a particular combination of sensors into the soap bar.

Combining information from the sensors enables reduced recording of false events, leading to reduced energy consumption, lower power and memory requirements, greater device efficiency and greater ease of data analysis. This is especially advantageous in consumer behaviour analysis settings, where data capture may be required over prolonged periods of time.

Such devices have improved simplicity and robustness which makes them practical to deploy over large populations.

Also, small low power devices are less likely to interfere with normal consumer activity, so the data collected is more representative of real behaviour.

### Summary of the Invention

The invention provides a system suitable for monitoring usage of a soap bar, the system comprising:
(a) a soap bar,
(b) a programmable data logger which is positioned in the soap bar, the data logger incorporating a motion sensor, a sound sensor, a data store for the logging of data and switching means for triggering the operation of the sound sensor in response to initial signals of soap motion generated by the motion sensor,
   and
(c) a data analysis device which is adapted to analyse data transmitted or acquired from the data logger to provide information about soap bar usage;
in which the duration of data logging is controllable in response to the signals received from the motion sensor and the sound sensor respectively,
and in which the data logger is programmed so that the data logging only takes place when the signals received from the sound sensor and the signals received from the motion sensor are each registered above pre-defined thresholds of duration or intensity.

The invention also provides a method for monitoring usage of a soap bar, including the steps of:
(a) acquiring data from a programmable data logger which is positioned in the soap bar, the data logger incorporating a motion sensor, a sound sensor, a data store for the logging of data and switching means for triggering the operation of the sound sensor in response to initial signals of soap motion generated by the motion sensor, and
(b) analysing the data so acquired to provide information about soap bar usage; characterised in that the operation of the sound sensor is triggered in response to initial signals of soap bar motion generated by the motion sensor, and in that the duration of data logging is controllable in response to the signals received from the motion sensor and the sound sensor respectively
and in which the data logger is programmed so that the data logging only takes place when the signals received from the sound sensor and the signals received from the motion sensor are each registered above pre-defined thresholds of duration or intensity.

### Detailed Description of the Invention

In the system and method of the invention, a programmable data logger is positioned in a soap bar. Generally the programmable data logger is at least partially embedded in the soap bar, for example in a hollow or cavity provided in the soap bar. Preferably the programmable data logger is fully embedded in the soap bar. In this way, consumer perceptibility (and therefore obtrusiveness) of the logger is minimised. The inventors have observed, unexpectedly, that the performance and functionality of the logger is not adversely affected to any significant extent in this environment.

The term "bar" in the context of the present invention generally means a solid unit of shape and size acceptable for use by human hands. In general, the bar will weigh between 70 and 250 g and have physical dimensions in excess of several cm in at least one dimension.

The bar will generally be of a hardness typical of toilet soaps (often characterised by the term "soft solid"). The hardness of a toilet soap bar may be expressed, for example, in "hardness units," which may be measured by a Penetrometer at room temperature (25°C). Each hardness unit is 1/10 mm. The hardness is represented by the depth travelled in 15 seconds into the bar by an ASTM D-1321 needle weighed down by a 50 gram weight. Three readings on an approximately 24-hour old bar are averaged. Acceptable toilet soap hardnesses usually range from 30 to 100 hardness units, preferably from 40 to about 75 units.

The term "soap" in the context of the present invention is intended to encompass any cleansing composition which removes soil from human skin and is suitable for application to the human body.

Accordingly a "soap bar" in the context of this invention will typically include one or more surfactants, and various additives and other optional ingredients as described further below.

Surfactants provide lather and assist in the removal of soil and germs.
Typically the surfactants are selected from anionic surfactants such as fatty acid soaps, synthetic anionic detergents, and mixtures thereof. By "fatty acid soap" is meant alkali metal or alkanolammonium salts of aliphatic alkane- or alkene monocarboxylic acids.

Examples of synthetic anionic detergents include the alkyl sulphates, alkyl ether sulphates, alkaryl sulphonates, alkanoyl isethionates, alkyl succinates, alkyl sulphosuccinates, alkyl ether sulphosuccinates, N-alkyl sarcosinates, alkyl phosphates, alkyl ether phosphates, and alkyl ether carboxylic acids and salts thereof, especially their sodium, magnesium, ammonium and mono-, di- and triethanolamine salts. The alkyl and acyl groups generally contain from 8 to 18, preferably from 10 to 16 carbon atoms and may be unsaturated. The alkyl ether sulphates, alkyl ether sulphosuccinates, alkyl ether phosphates and alkyl ether carboxylic acids and salts thereof may contain from 1 to 20 ethylene oxide or propylene oxide units per molecule.

Other classes of surfactant (such as nonionic, cationic or amphoteric surfactants and mixtures thereof) may also be included to boost bar properties such as foaming and/or skin mildness.

The total amount of surfactant(s) in the soap bar will usually range from about 20wt% to about 99wt%, preferably from about 30wt% to about 95wt%, more preferably from about 40wt% to about 90wt%, by total weight of surfactant based on the total weight of the soap bar.

A typical soap bar is based on fatty acid soaps (i.e. alkali metal or alkanolammonium salts of aliphatic alkane- or alkene monocarboxylic acids). A typical soap bar comprises from 70 to 85wt% of fatty acid soaps (by total weight fatty acid soap based on the total weight of the soap bar). In general sodium salts are used, although occasionally, 1 to 30wt% potassium or triethanolamine salts (by weight based on the total weight of the soap bar) are included in the formulation to increase the solubility of the soap and, hence, the bar's lathering properties.

Soap performance can be controlled through the proper blending of oils and fats to specific ratios, and the formation of the proper phase and colloidal structure. A typical blend is (a) tallow and/or palm oil with (b) coconut and/or palm kernel oil, generally in a ratio of (a):(b) between 85:15 and 50:50. Many soaps also contain harder oils such as palm stearines or softer oils such as soya bean.

Additionally, soap bars typically contain between 8 and 20wt% water, 0.5 to 1wt% salt (such as sodium chloride) and low levels (less than 5wt%) of glycerol. Both salt and glycerol modify the processability of soap during milling and plodding, as well as being carryover ingredients from the manufacture of the base soap.

Also, there are a variety of additives that may optionally be formulated into soap bars to provide additional consumer benefits or modify the performance of the products. Examples include free fatty acids, opacifiers (such as titanium dioxide and the like), fluorescers, dyes, chelants, antioxidants, lanolin, vitamin E, aloe vera gel, mineral oil, baking soda, inert materials (such as oatmeal, dextrin, starch, wax, talc and the like), cationic polymers based on natural materials such as cellulose, guar gums, and proteins, and skin antimicrobial agents (such as trichlorocarbanalide and trichlorohydroxydiphenyl ether).

In order that it is not adversely affected by moisture or direct water contact, the programmable data logger will generally be water-resistant. For optimum performance in the bathroom environment, the programmable data logger will most preferably be waterproof. To achieve this the programmable data logger may suitably be encapsulated with a water-impervious material to provide an Ingress Protection (IP) rating of IP67. Ingress Protection (IP) ratings are developed by the European Committee for Electro Technical Standardization (NEMA IEC 60529 Degrees of Protection Provided by Enclosures - IP Code), specifying the environmental protection the enclosure provides. A suitable encapsulation technique is injection moulding using a low pressure and low temperature technique and material.

The programmable data logger generally incorporates a power source, which is typically a DC power source such as a small (e.g. 3V) battery. To prolong battery life, a capacitor may be included. Alternatively the programmable data logger may be at least in part solar powered. As described above, the programmable data logger is most preferably encapsulated with a water-impervious material. In this case, it may be charged by induction coils provided in the programmable data logger and in a base unit.

The sound sensor incorporated in the programmable data logger is adapted to detect sounds associated with the use of the soap bar and to generate signals corresponding to the detected sounds. Typically, the sound sensor is an audio transducer, such as a microphone. Suitable microphones include contact microphones, otherwise known as pickup or piezo (piezo-electric) microphones, which are designed to transmit audio vibrations through solid objects.

Typically, the sound and/or vibrations which are detected by the sound sensor during use of the soap bar are filtered through a hardware filter on-board the programmable data logger, which is designed to capture the portion of the signal within a specified bandwidth which is indicative of actual washing events such as hand washing.

The motion sensor incorporated in the programmable data logger is adapted to detect motion of the soap bar and to generate signals corresponding to the detected motion. Typically, the motion sensor is an accelerometer capable of sensing accelerations along one or more axes. The accelerometer may be capable of sensing accelerations along three orthogonal axes. Any suitable triaxial accelerometer could be used or alternatively, two 2-axis ones mounted at right angles capable of sensing 3-dimensional acceleration data. Alternatively any other suitable motion sensor may be used. The motion sensor may be analogue or digital.

The data store incorporated in the programmable data logger preferably incorporates erasable non-volatile memory. Erasable (rewritable) memory allows re-use of the data logger. Non-volatile memory is preferable because this means the memory is protected in case of a loss of power (e.g. in the case of battery powered devices, when the battery loses power).

The data logger is programmable, and typically contains a programmable computing device, such as a microprocessor (e.g. a microcontroller) which is capable of reading and executing program instructions to control the various components within the soap bar usage monitoring system of the invention.

In a typical soap bar usage monitoring method using the system of the invention, the programmable data logger is first connected to a computer (typically a PC or similar). Programming will provide key operational parameters such as the activation and the duration of the data logging process.

The programmed data logger is then positioned in the bar of soap as described above.

The programmable data logger incorporates a motion sensor which is adapted to detect initial signals corresponding to soap bar motion. When such initial signals are detected, switching means trigger the operation of the sound sensor. Tilt switches are a preferred switching means in the context of the present invention in view of their small size, simple construction and low power consumption.

The data logger checks for motion of the soap bar, indicated by signals generated by the motion sensor (typically an accelerometer or similar). Data is also acquired from the sound sensor, activated by the initial detection of soap bar motion as described above.

The duration of data logging is controllable in response to the signals received from the motion sensor and the sound sensor respectively.

The inventors have found that the data acquired from the sound sensor provides a surprisingly reliable means of indicating whether an actual washing event (such as hand washing) is taking place, as opposed to merely shaking or moving the soap bar without contacting the skin (a false event).

Accordingly, in the system of the invention the data logger will typically be programmed so that data logging is started in response to the signals received from the motion sensor and the sound sensor. The data logger will also typically be programmed so that data logging only takes place when the signals received from the sound sensor and the signals received from the motion sensor are each registered above pre-defined thresholds of duration or intensity. In this way the system of the invention is able to operate in an "active data capture" mode for capturing the activity of interest, but is also able to revert to a "low power mode" at other times. This enables reduced recording of false events, leading to reduced energy consumption, lower power and memory requirements, greater device efficiency and greater ease of data analysis.

The data acquired by the system of the invention is analysed by a data analysis device to provide information about soap bar usage.

Typically, the data acquired by the system of the invention is downloaded to a remote data analysis device such as a computing device (e.g. a PC). Alternatively the data may be transmitted to the data analysis device by cellular telephony or wireless LAN technology, preferably "Wi-Fi" (wireless fidelity) enabling easy, fast communication via internet.

As described above, the programmable data logger is most preferably encapsulated with a water-impervious material. In this case, data reading may suitably be via IR.

The analysis can provide information about various aspects of soap bar usage, such as the duration of individual washing (e.g. hand washing) events, the frequency of individual washing (e.g. hand washing) events per day or a combination of the above.

A particular advantage of the system of the invention is its ability to identify different individual washing (e.g. hand washing) events, even when they are closely spaced apart in time, such as successive usages by family members.

Furthermore, data analysis can take place conveniently e.g. in a laboratory many miles away with minimal interference in the consumers' life.

### EXAMPLE

A programmable data logger as described above, incorporating *inter alia* a sound sensor (microphone) and a motion sensor (accelerometer), was embedded into a soap bar.

Two different events were recorded as follows:
(a) consumer walks with the soap, no actual washing usage
(b) consumer washes hands with the soap

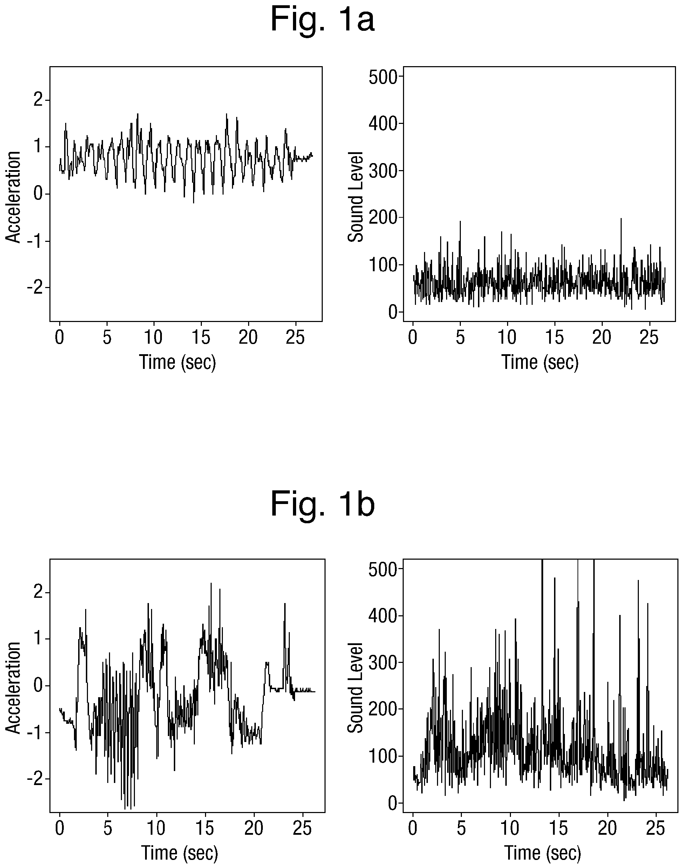

For (a): The appended **Figure 1a** illustrates the acceleration (left panel) and sound level (right panel) as measured by the embedded data logger.

For (b): The appended **Figure 1b** illustrates the acceleration (left panel) and sound level (right panel) as measured by the embedded data logger.

In the case of the false event (a), although there is sizeable variation in the acceleration, the sound level is bounded.

By contrast, in the case of the real event (b), both acceleration and sound signal consistently attain high values, such that this data is correctly identified as a washing event.

The sound signal therefore provides an accurate discriminator as to whether an actual washing event is taking place, as opposed to merely moving the soap bar without contacting the skin (a false event).

Using this information, the system of the invention can be set up to capture only the motion of interest. This is generally achieved by the tuning of hardware filters on board the programmable data logger, and by the design of the software algorithms controlling the data logging process.

## Claims

1. A system suitable for monitoring usage of a soap bar, the system comprising:
(a) a soap bar,
(b) a programmable data logger which is positioned in the soap bar, the data logger incorporating a motion sensor adapted to detect motion of the soap bar and to generate signals corresponding to the detected motion, a sound sensor adapted to detect sounds associated with the use of the soap bar and to generate signals corresponding to the detected sounds, a data store for the logging of data and switching means for triggering the operation of the sound sensor in response to initial signals of soap motion generated by the motion sensor,
and
(c) a data analysis device which is adapted to analyse data transmitted or acquired from the data logger to provide information about soap bar usage;
in which the duration of data logging is controllable in response to the signals received from the motion sensor and the sound sensor respectively,
and in which the data logger is programmed so that data logging only takes place when the signals received from the sound sensor and the signals received from the motion sensor are each registered above pre-defined thresholds of duration or intensity.

2. A system according to claim 1, in which the programmable data logger is at least partially embedded in a hollow or cavity provided in the soap bar.

3. A system according to claim 1 or 2, in which the sound sensor is an audio transducer such as a microphone.

4. A system according to any one of claims 1 to 3, in which the switching means is a tilt switch.

5. A system according to any one of claims 1 to 4, in which the data store incorporates erasable non-volatile memory.

6. A system according to any one of claims 1 to 5, in which the programmable data logger is programmed so that data logging is started in response to the signals received from the motion sensor and the sound sensor.

7. A system according to any one of claims 1 to 6, in which the soap bar comprises from 70 to 85wt% of fatty acid soaps (by total weight fatty acid soap based on the total weight of the soap bar).

8. A method for monitoring usage of a soap bar, including the steps of:
(a) acquiring data from a programmable data logger which is positioned in the soap bar, the data logger incorporating a motion sensor adapted to detect motion of the soap bar and to generate signals corresponding to the detected motion, a sound sensor adapted to detect sounds associated with the use of the soap bar and to generate signals corresponding to the detected sounds, a data store for the logging of data and switching means for triggering the operation of the sound sensor in response to initial signals of soap motion generated by the motion sensor,
and
(b) analysing the data so acquired to provide information about soap bar usage;
**characterised in that** the operation of the sound sensor is triggered in response to initial signals of soap bar motion generated by the motion sensor, and **in that** the duration of data logging is controllable in response to the signals received from the motion sensor and the sound sensor respectively
and in which the data logger is programmed so that data logging only takes place when the signals received from the sound sensor and the signals received from the motion sensor are each registered above pre-defined thresholds of duration or intensity.

## Patentansprüche

1. System, das die Verwendung eines Seifenstücks überwachen kann, wobei das System Folgendes enthält:
(a) ein Seifenstück,
(b) eine programmierbare Datenregistriereinrichtung, die in dem Seifenstück positioniert ist, wobei die Datenregistriereinrichtung einen Bewegungssensor, der ausgelegt ist, eine Bewegung des Seifenstücks zu detektieren und Signale, die der detektierten Bewegung entsprechen, zu erzeugen, einen Schallsensor, der ausgelegt ist, Schalle, die der Verwendung des Seifenstücks zugeordnet sind, zu detektieren und Signale zu erzeugen, die den detektierten Schallen entsprechen, einen Datenspeicher zum Registrieren von Daten und ein Schaltmittel, um den Betrieb des Schallsensors als Antwort auf anfängliche Signale einer Seifenbewegung, die durch den Bewegungssensor erzeugt werden, auszulösen, umfasst, und
(c) eine Datenanalyseeinrichtung, die ausgelegt ist, Daten, die von der Datenregistriereinrichtung gesendet oder erfasst wurden, zu analysieren, um Informationen über die Verwendung des Seifenstücks bereitzustellen; wobei
die Dauer der Datenregistrierung als Antwort auf die Signale, die vom Bewegungssensor bzw. vom Schallsensor empfangen werden, steuerbar ist und
die Datenregistriereinrichtung derart programmiert ist, dass eine Datenregistrierung nur stattfindet, wenn die Signale, die vom Schallsensor empfangen werden, und die Signale, die vom Bewegungssensor empfangen werden, jeweils über vordefinierten Schwellenwerten der Dauer oder der Intensität registriert werden.

2. System nach Anspruch 1, wobei die programmierbare Datenregistriereinrichtung mindestens teilweise in eine Mulde oder einen Hohlraum, die oder der im Seifenstück vorgesehen ist, eingebettet ist.

3. System nach Anspruch 1 oder 2, wobei der Schallsender ein Audiowandler wie z. B. ein Mikrophon ist.

4. System nach irgendeinem der Ansprüche 1 bis 3, wobei das Schaltmittel ein Neigungsschalter ist.

5. System nach irgendeinem der Ansprüche 1 bis 4, wobei der Datenspeicher löschbaren nichtflüchtigen Speicher umfasst.

6. System nach irgendeinem der Ansprüche 1 bis 5, wobei die programmierbare Datenregistriereinrichtung derart programmiert ist, dass eine Datenregistrierung als Antwort auf die Signale, die vom Bewegungssensor und vom Schallsensor empfangen werden, gestartet wird.

7. System nach irgendeinem der Ansprüche 1 bis 6, wobei das Seifenstück im Bereich von 70 bis 85 Gew.-% Fettsäureseifen (bezogen auf das Gesamtgewicht von Fettsäurenseife auf der Grundlage des Gesamtgewichts des Seifenstücks) enthält.

8. Verfahren, um die Verwendung eines Seifenstücks zu überwachen, das die folgenden Schritte umfasst:
(a) Erfassen von Daten von einer programmierbaren Datenregistriereinrichtung, die in dem Seifenstück positioniert ist, wobei die Datenregistriereinrichtung einen Bewegungssensor, der ausgelegt ist, eine Bewegung des Seifenstücks zu detektieren und Signale, die der detektierten Bewegung entsprechen, zu erzeugen, einen Schallsensor, der ausgelegt ist, Schalle, die der Verwendung des Seifenstücks zugeordnet sind, zu detektieren, und Signale zu erzeugen, die den detektierten Schallen entsprechen, einen Datenspeicher zum Registrieren von Daten und ein Schaltmittel, um den Betrieb des Schallsensors als Antwort auf anfängliche Signale einer Seifenbewegung, die durch den Bewegungssensor erzeugt werden, auszulösen, umfasst, und
(b) Analysieren der Daten, die auf diese Weise erfasst wurden, um Informationen über die Verwendung des Seifenstücks bereitzustellen;
**dadurch gekennzeichnet, dass** der Betrieb des Schallsensors als Antwort auf anfängliche Signale einer Seifenbewegung, die durch den Bewegungssensor erzeugt werden, ausgelöst wird und dass die Dauer der Datenregistrierung als Antwort auf die Signale, die vom Bewegungssensor bzw. vom Schallsensor empfangen werden, steuerbar ist, wobei
die Datenregistriereinrichtung derart programmiert ist, dass eine Datenregistrierung nur stattfindet, wenn die Signale, die vom Schallsensor empfangen werden, und die Signale, die vom Bewegungssensor empfangen werden, jeweils über vordefinierten Schwellenwerten der Dauer oder der Intensität registriert werden.

## Revendications

1. Système approprié pour surveiller l'utilisation d'une barre de savon, le système comprenant:
(a) une barre de savon,
(b) un enregistreur de données programmable qui est positionné dans la barre de savon, l'enregistreur de données incorporant un détecteur de mouvement adapté pour détecter un mouvement de la barre de savon et pour émettre des signaux correspondant au mouvement détecté, un détecteur de sons adapté pour détecter les sons associés avec l'utilisation de la barre de savon et pour émettre des signaux correspondant aux sons détectés, un stockage de données pour l'enregistrement des données et un moyen de commutation pour déclencher le fonctionnement du détecteur de sons en réponse à des signaux initiaux de mouvement du savon émis par le détecteur de mouvement,
et
(c) un dispositif d'analyse de données qui est adapté pour analyser les données transmises ou acquises par l'enregistreur de données pour fournir des informations concernant l'utilisation de la barre de savon ;
dans lequel la durée d'enregistrement des données est régulable en réponse aux signaux reçus du détecteur de mouvement et du détecteur de sons respectivement,
et dans lequel l'enregistreur de données est programmé de telle sorte que l'enregistrement des données n'a lieu que quand les signaux reçus du détecteur de sons et les signaux reçus du détecteur de mouvement sont repérés chacun au-dessus de seuils de durée ou d'intensité prédéfinis.

2. Système selon la revendication 1, dans lequel l'enregistreur de données programmable est inclus au moins en partie dans un creux ou cavité prévu dans la barre de savon.

3. Système selon la revendication 1 ou 2, dans lequel le détecteur de sons est un transducteur audio comme un microphone.

4. Système selon l'une quelconque des revendications 1 à 3, dans lequel le moyen de commutation est un commutateur pivotant.

5. Système selon l'une quelconque des revendications 1 à 4, dans lequel le stockage de données incorpore une mémoire non volatile effaçable.

6. Système selon l'une quelconque des revendications 1 à 5, dans lequel l'enregistreur de données programmable est programmé de telle sorte que l'enregistrement des données est commencé en réponse aux signaux reçus du détecteur de mouvement et du détecteur de sons.

7. Système selon l'une quelconque des revendications 1 à 6, dans lequel la barre de savon comprend de 70 à 85 % en poids de savons d'acides gras (en poids total de savon d'acides gras sur la base du poids total de la barre de savon).

8. Procédé pour surveiller l'utilisation d'une barre de savon, incluant les étapes de :
(a) acquisition de données à partir d'un enregistreur de données programmable qui est positionné dans la barre de savon, l'enregistreur de données incorporant un détecteur de mouvement adapté pour détecter un mouvement de la barre de savon et pour émettre des signaux correspondant au mouvement détecté, un détecteur de sons adapté pour détecter les sons associés avec l'utilisation de la barre de savon et pour émettre des signaux correspondant aux sons détectés, un stockage de données pour l'enregistrement des données et un moyen de commutation pour déclencher le fonctionnement du détecteur de sons en réponse à des signaux initiaux de mouvement du savon émis par le détecteur de mouvement,
et
(b) analyse des données ainsi acquises pour fournir des informations concernant l'utilisation de la barre de savon ;
**caractérisé en ce que** le fonctionnement du détecteur de sons est déclenché en réponse à des signaux initiaux de mouvement de la barre de savon émis par le détecteur de mouvement, et **en ce que** la durée d'enregistrement des données est régulable en réponse aux signaux reçus du détecteur de mouvement et du détecteur de sons respectivement
et dans lequel l'enregistreur de données est programmé de telle sorte que l'enregistrement des données n'a lieu que quand les signaux reçus du détecteur de sons et les signaux reçus du détecteur de mouvement sont repérés chacun au-dessus de seuils de durée ou d'intensité prédéfinis.
